# EUROPEAN PATENT SPECIFICATION

(11) **EP 4 366 612 B1**
(45) Date of publication and mention of the grant of the patent: **02.09.2026**
(21) Application number: 22751436.1
(22) Date of filing: 01.07.2022
(51) Int. Cl.: A61B 5/024, A61B 5/1171, A61B 5/00

(54) **SENSING SYSTEM AND METHOD FOR SMART RINGS EMPLOYING SENSOR SPATIAL DIVERSITY**
SENSORISCHES SYSTEM UND VERFAHREN FÜR INTELLIGENTE RINGE MIT RÄUMLICHER VIELFALT DER SENSOREN
SYSTÈME ET MÉTHODE DE DÉTECTION POUR ANNEAUX INTELLIGENTS UTILISANT LA DIVERSITÉ SPATIALE DES CAPTEURS

(30) Priority: 06.07.2021 US 202163218736 P
(43) Date of publication of application: 15.05.2024
(73) Proprietor: Senbiosys, 2000 Neuchâtel (CH)
(72) Inventor: CAIZZONE, Antonino, 2014 Milvignes (CH); BOUKHAYMA, Assim, 2000 Neuchâtel (CH); BARISON, Anthony, 2000 Neuchâtel (CH)
(74) Representative: HGF
(86) International application number: PCT/IB2022/056156
(87) International publication number: WO 2023/281370

(56) References cited:
- US-A1- 2015 018 647
- US-A1- 2017 337 413
- US-A1- 2020 000 345
- CAIZZONE ANTONINO ET AL: "A 2.6 $\mu$W Monolithic CMOS Photoplethysmographic (PPG) Sensor Operating With 2 $\mu$W LED Power for Continuous Health Monitoring", IEEE TRANSACTIONS ON BIOMEDICAL CIRCUITS AND SYSTEMS, IEEE, US, vol. 13, no. 6, 1 December 2019 (2019-12-01), pages 1243 - 1253, XP011763620, ISSN: 1932-4545, [retrieved on 20200101], DOI: 10.1109/TBCAS.2019.2944393
- KHAN YASSER ET AL: "Organic Multi-Channel Optoelectronic Sensors for Wearable Health Monitoring", IEEE ACCESS, vol. 7, 6 September 2019 (2019-09-06), pages 128114 - 128124, XP011746500, DOI: 10.1109/ACCESS.2019.2939798

## Description

### RELATED APPLICATIONS

This application claims the benefit under 35 USC 119(e) of U.S. Provisional Application No. 63/218,736, filed on July 6, 2021.

### BACKGROUND OF THE INVENTION

Nowadays, wearable devices, such as fitness trackers or smartwatches, with optical heart rate sensors, are becoming common. Even rings are now available with these sensors.

The technology behind these sensors is called photoplethysmography (PPG), which is an optical measurement technique used to detect blood volume changes in living tissues. A PPG sensor requires a few optoelectronics components, such as a light source, e.g. light-emitting-diode (LED), to illuminate the living tissue, a photodetector (PD) to track any light intensity variation due to the blood volume change, and an analog front-end (AFE) for signal conditioning and processing. Today, the importance of PPG for medical monitoring is proven by the number of primary vital signs directly or indirectly that can be resolved by it.

In a typical example, the PPG signal is obtained by shining light from the LED at a given wavelength, in the visible or near-infrared range, into the finger, wrist, forehead, or ear lobes. The PPG sensor's photodetector detects the light transmitted through (transmissive PPG) or reflected (reflective PPG) from the tissue and transforms it into a photogenerated current. The detected signal, i.e. PPG signal, has two different components: a large DC (quasi-static) component corresponding to the light diffusion through tissues and non-pulsatile blood layers, and a small AC (pulsatile) part due to the diffusion through the arterial blood. The AC component is only a very small fraction (typically 0.2% to 2%) of the DC one, meaning the AC component is 500 to 50 times smaller than the DC component. This AC/DC ratio mostly depends on the sensor's location on the body, and the LED wavelength, and weakly on the skin tone. This AC/DC ratio is often called perfusion-index (PI) and ultimately sets one of the challenges for any PPG readout system. Indeed, the AC component carries most of the biomedical information. Low PI values lead to reduced signal fidelity, complicated signal processing schemes and larger power consumption. US 2020/000345 A1 discloses a wearable ring of optical biometric sensors for measuring biometric parameters. US 2017/337413 A1 discloses a bio-sensor device comprising a light source and an array of photodetectors for determining a characteristic of a body part. "A 2.6uW Monolithic CMOS Photoplethysmographic (PPG) sensor operating with 2uW LED power for continuous health monitoring" by Caizzone et al discloses a PPG sensor integrating an array of dedicated pinned-photodiodes (PPD). "Organic multichannel optoelectronic sensors for wearable health monitoring" by Khan et al discloses a wearable two-channel PPG sensor.

### SUMMARY OF THE INVENTION

The invention is defined in the appended set of claims.

### SUMMARY OF THE DISCLOSURE

This disclosure relates to the ever-growing field of the health monitoring and remote identification. It concerns devices and operating methods allowing on one hand a better photoplethysmographic sensing on the finger or other digit or other location where transmissive PPG and/or reflective PPG is possible. It can enable lower power consumption, higher fidelity, and/or greater versatility to different use cases and users' specificities. Photoplethysmographic sensing enables monitoring of characteristics such as heart rate (HR), its variability (HRV), the oxygen saturation (SpO2), the breathing rate (BR) and the blood pressure (BP). On the other hand, it combines the health monitoring functions with user-identification capabilities, the latter taking direct or indirect advantage of the former.

In examples, the PPG system takes advantage of sensor spatial diversity to enhance the quality and the reliability of the PPG measurements in smart rings, for example. Indeed, there are many possible use cases related to the PPG sensing in the finger, each of them coming with different engineering constraints. Moreover, finger PPG signals show excellent signal fidelity. Nevertheless, these signals can also suffer from extremely low perfusion under hypothermia or heavy motion artifacts. Engineering a PPG sensor for a smart ring no matter its use case is very challenging and may become a limiting factor for the adoption of PPG sensors in smart rings. At the same time, a smart ring can be a good place for remote identification, which can directly or indirectly take advantage of the PPG sensors. The user-identification can help extend the possible use cases of a smart ring, well beyond wellness/lifestyle one.

As mentioned, the (sensing) body location of the PPG sensor affects the PI and the signal quality. Different measurement sites can be used including the wrist, the finger and the ear region (both the lobe and the canal). The finger, however, has been shown to be among the best locations in terms of PI, at a given optical power, providing better biomedical sensing.

In addition to the intrinsically larger PI, the finger is the standard location when it comes to oxygen saturation monitoring in medical environments, which is based on finger clips using the transmissive PPG method.

If on one hand, wearable devices usually embed reflective PPG thanks to its intrinsic lower power consumption and easiness to place on the body, on the other hand the transmissive method remains the standard for accurate oxygen saturation analysis. Indeed, reflective PPG can be affected by many factors, including reduced blood perfusion due to hypothermia (cold fingers), applied pressure and motion artefacts (MA). MA can be of various types and ultimately distort the PPG signal setting a challenge for PPG systems. MA can be periodic or non-periodic and can present a much larger amplitude than the AC component of the PPG signal. In addition, MA can fall within the same frequency band as the HR. For these reasons, MA needs to be minimized and/or compensated as much as possible.

The present approach integrates multiple PPG sensors around a smart ring. This leads to spatial diversity (or distribution) and results in different PPG channels combining transmissive and reflective PPG, in the same device. The redundancy of such a set-up enables the present system to adapt to the particular use case and any user idiosyncrasies. Indeed, the arterial/venous pattern is specific to each user. In addition, the spatial diversity can effectively reduce the impact of both hypothermia and motion artefacts and ultimately reduce the effect of badly placed PPG sensors. This is particularly important on the finger, since the limbs and digits tend to exhibit substantial motion artefacts, particularly during sports.

Identification by means of wearable devices is getting more and more popular with the ever-growing exposure to digitalization. Digitalization imposes stricter measures for users' identification, particularly when the device is used for accessing personal environments, including, but not limited to, financials or private assets. With a smart ring, the usual identification based on fingerprints, face recognition and retina/iris inspection can be complemented with the information coming from PPG sensors. Indeed, similar to a retina scanning system, the arterials/venous pattern at the level of the finger is specific to the user and this information can be very useful in enhancing the security of the wearable identification.

In general, according to one aspect, the disclosure features a ring system, comprising sensors for performing both transmissive and reflective photoplethysmographic sensing of a user's finger on which the ring system is worn.

Typically, some or each of the sensors performs reflective photoplethysmographic sensing and transmissive photoplethysmographic sensing in conjunction with another one of the sensors.

In general, according to another aspect, the disclosure features a ring system comprising sensors each performing photoplethysmographic sensing of a user's finger on which the ring system is worn.

In general, according to another aspect, the disclosure features a ring system comprising sensors each performing reflective photoplethysmographic sensing of a user's finger on which the ring system is worn.

In examples, the ring systems perform user identification based on information from the photoplethysmographic sensing.

In examples, the ring systems can comprise at least 4 of the sensors.

In examples, the ring systems can further comprise 8 or more of the sensors.

In examples, each of the sensors can comprise photodiodes and light emitters.

In examples, each of the sensors can comprise a two dimensional array of photodiodes and light emitters.

In examples, the sensors can comprise an annular photodiode array and an annular light emitter array.

In some implementations, the light emitters are organic light emitting diodes and the photodiode array includes organic photodiodes.

The ring system further comprises a control unit for triggering the different sensors to perform reflective photoplethysmographic sensing and transmissive photoplethysmographic sensing. The control unit is configured to use results from the transmissive photoplethysmographic sensing to validate the reflective photoplethysmographic sensing. Further, the control unit might be configured to determine which of the sensors to use for ongoing reflective photoplethysmographic sensing.

The control unit can also use the sensors around the ring to track arterial and/or venous patterns in connection with user identification.

A sensor block having inertial sensors and/or a user temperature sensor and an ambient temperature sensor is helpful in some examples.

### BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:
Fig. 1 is a side cross-sectional view perpendicular to the axis of a sensor ring system, according to the invention;
Fig. 2 is a side cross-sectional view perpendicular to the axis of a sensor ring system, according to a second embodiment of the invention;
Fig. 3 is a schematic plan view of a PPG sensor 200;
Fig. 4 is a side cross-sectional view perpendicular to the axis of a sensor ring system, according to a third embodiment of the invention;
Fig. 5 is partial view looking outward along a radius of the sensor ring of the third embodiment showing the PPG sensor subsystem PPGR on the ring's PCB;
Fig. 6 is a flow diagram showing the operation of the control unit;
Figs. 7A, 7B, and 7C are schematic views showing the transmissive PPG readings between the different sensor units;
Fig. 8 is a schematic view showing a comprehensive set of transmissive PPG readings between sensor units in both directions;
Fig. 9 is a schematic view of user identification; and
Fig. 10 shows signal analysis employed for user identification by the control unit.

### DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

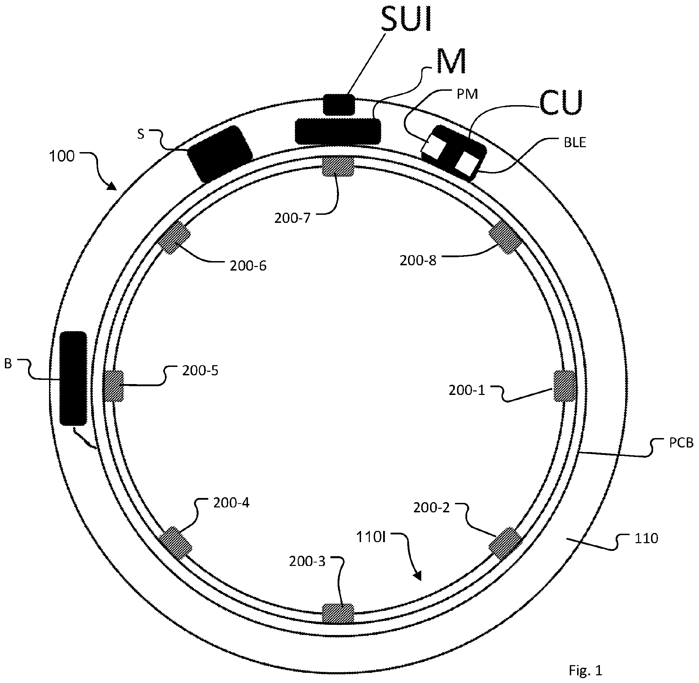

Fig. 1 shows a first embodiment of the sensor ring system 100.

In general, the sensor ring includes a ring or annual-shaped ring body 110 having an inner bore 110I sized to receive a human digit such as a finger, and specifically an index finger, middle finger, ring finger, or little finger or pinkie. The ring body 110 is typically a molded plastic or ceramic material, but could also be metal.

An array of PPG sensor units 200-1 to 200-8 are distributed around the inner bore and directed inwardly in order to perform PPG sensing of the digit inserted into the inner bore 110I. In the illustrated example, the sensor units 200-1 to 200-8 are evenly arrayed in a concentric ring.

A control unit CU is installed on a ring-shaped, concentric printed circuit board PCB. The control unit CU controls the triggering of and readout from the PPG sensors 200. In one mode of operation, the control unit CU alternates between a transmissive PPG mode and reflective PPG mode. The control unit CU preferably includes a Bluetooth-low-energy communications block BLE and a power management block PM, for communicating data with the external world and regulating system power, respectively.

The control unit CU stores sensor readings to a memory unit M, which also contains the operating instructions for the system set-up and other firmware components required for operation.

A battery B provides power to the system via the PCB, and is generally controlled by the power management unit PM in order to provide for extended operation.

A sensor block S has inertial sensors, including a three axis accelerometer and a three axis gyroscope. In some cases, the accelerometer and the gyroscope each have more than three axes, possibly six axes. In addition, the sensor block S also preferably has a user temperature sensor and an ambient temperature sensor, for separate body and environmental temperature detection.

A smart user interface block SUI provides active feedback from the device to the user and also receives user control input. In some examples, the SUI block includes a visible LED array and a touch sensor system, such as a capacitive touch sensor array.

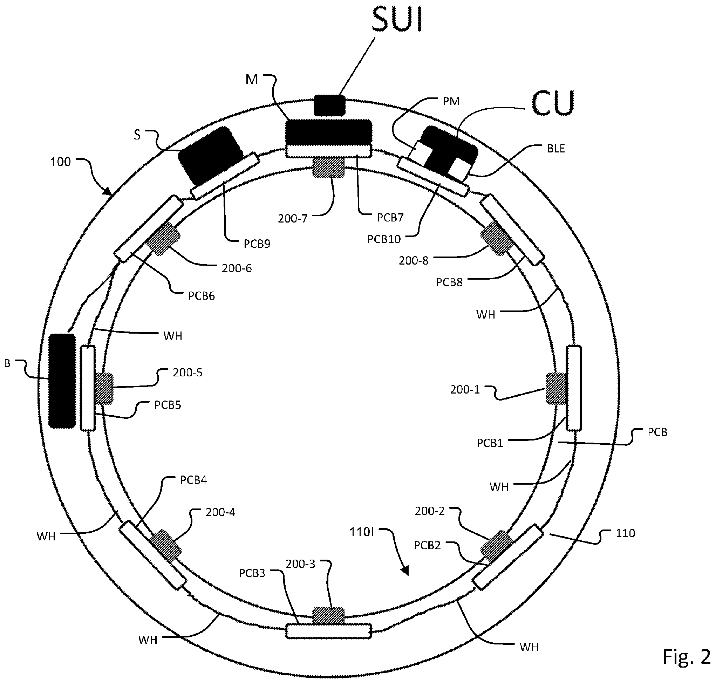

Fig. 2 shows a second embodiment of the sensor ring system 100. This uses a series of small independent PCBs PCB1-PCB10 for each of the sensor units 200, the battery B, the memory and smart user interface block SUI, the control unit CU, and sensor block S. Each of these PCBs are interconnected with wiring harnesses WH in a daisy-chained fashion.

In still other examples, the independent PCBs are replaced with one or more flexible PCBs. In both cases, flexible PCBs can be used to allow a better integration into the system.

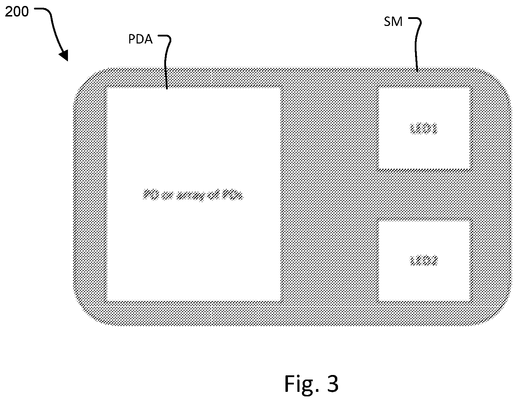

Fig. 3 shows an exemplary configuration for the PPG sensors 200.

In a typical implementation, each PPG sensor 200 includes at least one and preferably multiple photodiodes. In one example, an array of at least 8 by 8 photodiodesPDA is provided on a submount SM.

Each sensor 200 also preferably includes one or more light emitters such as LEDs or vertical cavity surface emitting lasers (VCSELs) installed on the submount SM, next to the photodiode array PDA. Moreover, the light emitters can operate at different wavelengths. In the illustrated example, two LEDs LED1 and LED2 are provided on the submount SM that emit at different wavelength in the visible and/or infrared.

In a current example, to reduce the PPG sensor size, the PPG readout electronics is embedded into the array of photo diodes PDA.

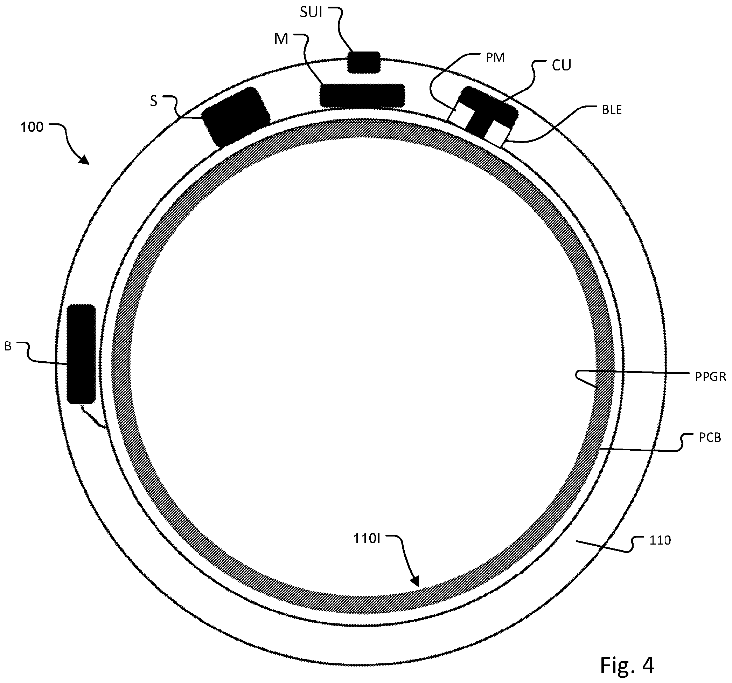

Fig. 4 shows a third embodiment of the sensor ring system 100.

This example includes a flexible and/or circular PCB, supporting an annular PPG sensor subsystem PPGR that follows the curvature of the ring's inner bore 110I.

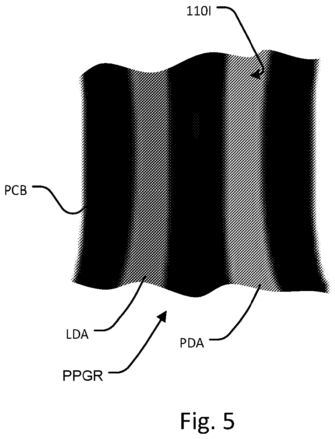

As shown in Fig. 5, the PPG sensor subsystem PPGR includes a circular and curved, annular, photodiode array PDA and a circular and curved, annular, LED array LDA on the inner bore 1101 of the ring system. The photodiode array PDA and the LED array LDA are directed inward toward the axis of the ring and arranged next to each other along the axial direction of the ring system 100.

To achieve a truly curved optoelectronic design, some embodiments use flexible organic photodiodes in the photodiode array PDA and organic LEDs (OLEDs) in the laser diode array LDA. Indeed, today's organic optoelectronics elements provide performance closer and closer to silicon ones, with the advantage of more versality, reduced power consumption and lower costs.

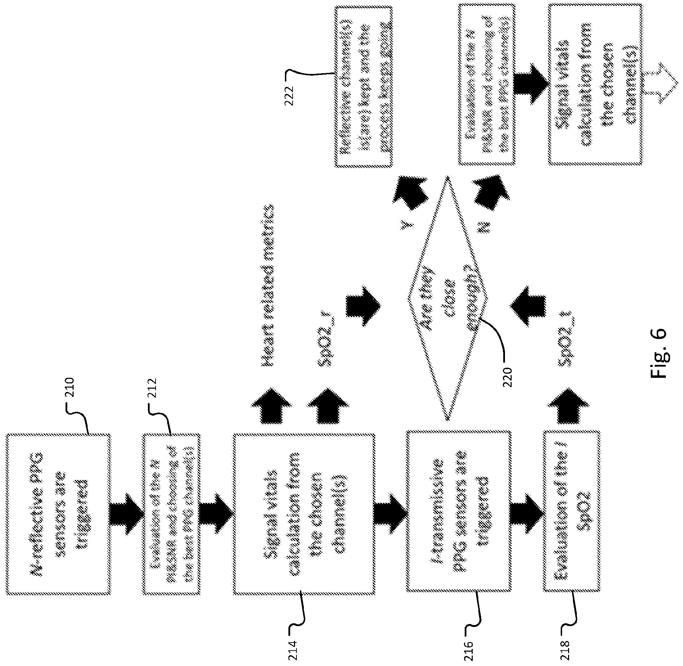

Fig. 6 is a flow diagram showing one method of operation of the ring system 100. This enables the control unit CU to optimize the PPG signal quality and light source power consumption.

In more detail, in step 210, once the ring system 100 is correctly worn as determined by the control unit CU, it triggers all the PPG sensors 210 consecutively in reflective mode. That is, each sensor's light emitters are activated while performing PPG sensing with the sensor's photodiodes.

Then, in step 212, the control unit calculates the perfusion index (PI) and the signal-to-noise ratio (SNR) of the PPG signals for each PPG sensor 200 and the best channel or PPG sensor 200 with the best PI and SNR is stored kept in the memory M. The control unit CU also stores in memory the set-up, per each reflective PPG channel, allowing the best signal quality, namely, but not limited to, the LED driving current, sampling frequency, PPG sensor integration time, gain and resolution.

If more than one PPG sensor 200 provides good PI and SNR, then more than one PPG reflective channel is stored with the corresponding set-up. The response of these multiple good PPG sensors can then be voted or fused to enhance the quality of the readings.

From the chosen channel or combination of channels, the signal vitals, including - but not limited to - the heart rate HR and the blood oxygen saturation SpO2 based on the reflective mode SPO2_r, are computed by the control unit CU in step 214 as part of the PPG sensing. In the case of a combination of channels, the vitals can be fused or voted from each independent channel.

Then, for the transmissive mode SpO2, the control unit CU triggers, with a given repetition frequency, three transmissive PPG recordings in step 216. In other examples, more than three transmissive PPG recordings is performed such as six or 16 or more with the eight sensor unit arrangement shown.

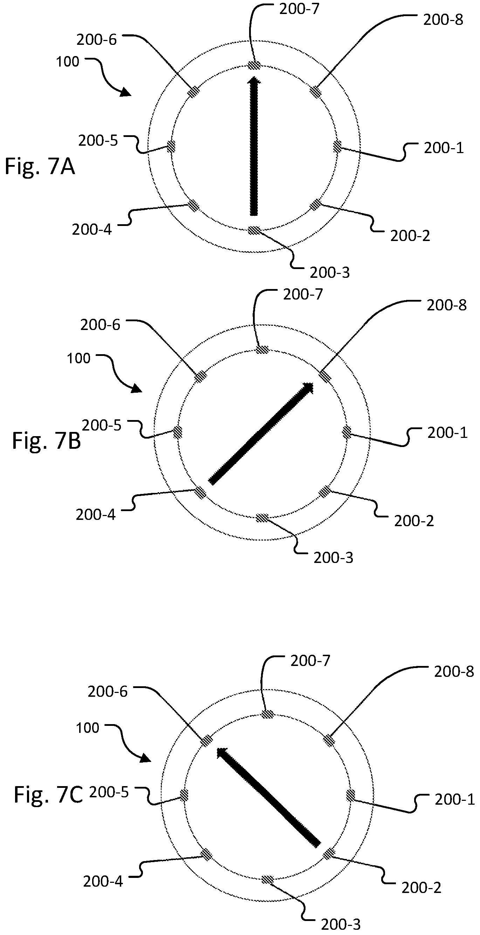

Figs 7A, 7B, 7C are schematic illustrations showing several transmissive PPG sensing operations, namely south-north (SN), south-east (SE) and south-west (SW).

In some examples, transmissive PPG sampling operations are performed between every pair of sensors 200 in both directions for each pair.

Returning to Fig. 6, the SpO2 is computed out of each of the transmissive PPG channels in step 218. The three channels are fused or voted, and the final value is kept in memory, as a calibration value, namely SpO2_t.

If the three transmissive PPG channels fail to provide a reference value, for instance in the case the fusion or the voting is impossible because of too much discrepancy between them, then step 216 is repeated in the opposite lighting direction, namely north-south (NS), north-east (NE) and north-west (NW), in one example.

In step 220, the SpO2 value computed from the reflective channel as in step 214, namely SpO2_r, is compared with the calibration value, i.e. SpO2_t, out of the transmissive channel. It is then determined if the two SpO2 values, i.e. SpO2_t and SpO2_r, are within a given range difference.

If SpO2_t and SpO2_r are within the range difference, then the reflective channel (or combination of channels) is kept in step 222.

On the other hand, motion artefacts are detected, by the means of the sensor S, the control unit CU reacts by increasing the repetition frequency, resulting into more frequent checks versus the calibration value.

The smart ring can also be operated in transmissive mode only. In this case the control unit CU operates the ring and the PPG sensors in the following way.

At a given repetition frequency, three transmissive PPG recordings are triggered, namely south-north (SN), south-east (SE) and south-west (SW).

Then, the SpO2 is computed out of each of the transmissive PPG channels. The three channels are fused or voted, and the final value is kept in memory, as the reference value, namely SpO2.

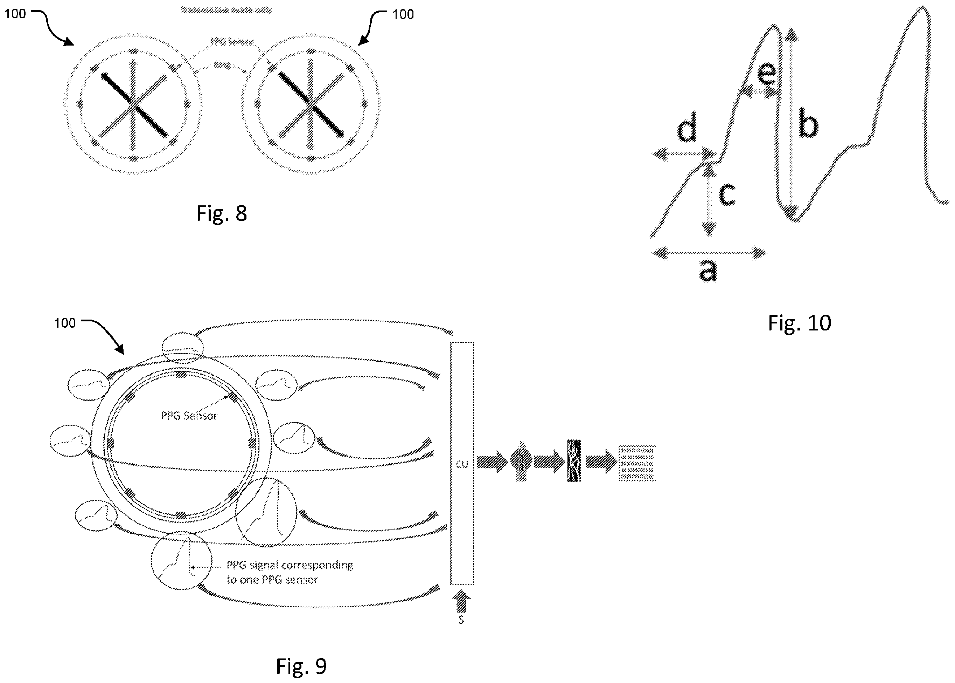

If the three transmissive PPG channels fail to provide a reference value, for instance in the case the fusion or the voting is impossible because of too much discrepancy between them, then the step is repeated in the opposite lighting direction, namely north-south (NS), north-east (NE) and north-west (NW), as in Fig. 8.

The user interface SUI block allows the exchange of information between the user and the device. On one hand, the SUI, by the means of the integrated LED, provides optical feedback to the user, for instance, if the device is used for fitness purposes. The LED can shine in green, orange, and red depending on the cardiac zones of the user. At the same time, the user can, by the means of the integrated touch sensor, trigger a particular training function or a meditative phase.

Fig. 9 shows how the spatial distribution of the PPG sensors around the ring allows tracking of the user's venous pattern, very similar to what conceptually can be done by an iris scanner. Indeed, the control unit CU analyzes for each n reflective path the type of corresponding PPG signal. In addition, each transmissive path is also assessed in some examples. By fusing these signals and the corresponding information, the arterial and/or venous pattern is deduced by the control unit CU, indirectly, without the need of further hardware.

Unlike a standard venous recognition system which utilizes IR-based cameras, the present embodiment utilizes the generated PPG signals out of the reflective based PPG sensors. All the n PPG signals are detected out of the n PPG sensors. The n PPG signals are fed to the control unit CU which has stored in memory the expected user's venous pattern under the form of PPG temporal and spatial features.

Fig. 10 shows the spatial features.

In addition to the above-mentioned figure, the PPG PI and SNR are also used, since a larger SNR or a larger PI result from a richer venous/arterial distribution. The spherical distribution of the PPG sensors allows the PPG signals to integrate the maximum possible signal features a, b, c, d, e leading to more robust identification. If an identification by means of this manner is not possible, then the user interface SUI, which is equipped with a touch sensor, is used in this regard, by analyzing the fingerprint of another finger.

The sensor block S integrates a temperature sensor which analyzes the ambient and the finger temperature. This is used for two main reasons: accounting for the vasodilation and vasoconstriction in the identification process. Indeed, both can temporarily bias the venous pattern, so the temperature information helps the control unit CU calibrating the identification in the best way.

Providing continuous temperature readings of the user and its environment helps scout diseases like covid-19, on one hand, and on the other hand, enhances quality of life and sleep.

Integrating a plurality of PPG sensors onto a ring system comes with the advantage of creating a sensing platform tailored to the user's biomedical specificities. In essence, each user has his/her own arterial pattern, and this must be considered to reduce the system power consumption and enhance signal quality. Very often wearable devices, including smart rings, lack this functionality and this results in using more power to chase good biometric signals, eventually limiting their use cases and the user's experience. In addition, the spatial distribution of the PPG sensors allows one device to be used in both reflective and transmissive modes, expanding the possible use cases beyond wellness/lifestyle.

Moreover, the present embodiments do not need user intervention to find a good sensing spot in the ring. Indeed, spatial diversity guarantees a scanning of the finger itself resulting in a user specific configuration. This is particularly important at night, since smart rings tend to move around the finger without any action from the user, which can lead to losing a good sensing spot.

The plurality of PPG sensors can also be used for identification purposes, since, by means of some features analysis, the PPG signals can be used for deducing the user's arterial/venous pattern.

The obvious industrial applications relate to the growing business of smart rings. This is an emerging market which is just showing a small fraction of its future potential. This is particularly true in regard to the growing use of devices for monitoring sleep and covid-19 outbreaks. The proposed embodiment and method are particularly interesting in this regard, due to the challenges resulting from effectively measuring a PPG signal in the finger for very different use cases. In addition, using the device for identification purposes can expand the market share of a device like this in contrast to more mature wearables, like smartwatches, which benefit from huge market penetration.

## Claims

1. A ring system (100), comprising
sensors (200) configured to perform both transmissive and reflective photoplethysmographic sensing of a user's finger on which the ring system is worn; and
a control unit configured to trigger the different sensors to perform reflective photoplethysmographic sensing and transmissive photoplethysmographic sensing; and
**characterized in that** the control unit is configured to use results from the transmissive photoplethysmographic to validate the reflective photoplethysmographic sensing.

2. The ring system (100) according to claim 1,
wherein the sensors are each configured to perform reflective photoplethysmographic sensing and transmissive photoplethysmographic sensing in conjunction with another one of the sensors.

3. The ring system (100) according to claim 1, wherein
the sensors (200) each are configured to perform reflective photoplethysmographic sensing of a user's finger on which the ring system is worn.

4. The ring system (100) according to any of claims 1-3, wherein the ring system is configured to perform user identification based on information from the photoplethysmographic sensing.

5. The ring system (100) according to any of claims 1-4, comprising at least 4 of the sensors (200).

6. The ring system (100) according to any of claims 1-5, comprising 8 or more of the sensors (200).

7. The ring system (100) according to any of claims 1-6, wherein each of the sensors (200) comprise photodiodes and light emitters.

8. The ring system (100) according to any of claims 1-7, wherein each of the sensors (200) comprises a two dimensional array of photodiodes and light emitters.

9. The ring system (100) according to any of claims 1-8, wherein the sensors (200) comprise an annular photodiode array and an annular light emitter array.

10. The ring system (100) according to claim 9, wherein the light emitters are organic light emitting diodes and the photo diode array are organic photodiodes.

11. The ring system (100) according to claim 1, wherein the control unit is configured to use results from the transmissive photoplethysmographic to determine which of the sensors (200) to use for ongoing reflective photoplethysmographic sensing.

12. The ring system (100) according to any of claims 1-11, wherein the control unit is configured to use the sensors (200) around the ring to track arterial and/or venous patterns.

13. The ring system (100) according to any of claims 1-12, further comprising a sensor block having inertial sensors.

14. The ring system (100) according to claim 13, wherein the sensor block includes a user temperature sensor and an ambient temperature sensor.

## Patentansprüche

1. Ringsystem (100), umfassend
Sensoren (200), die dazu eingerichtet sind, sowohl eine transmissive als auch eine reflektive photoplethysmographische Messung an einem Finger eines Benutzers durchzuführen, an dem das Ringsystem getragen wird; und
eine Steuereinheit, die dazu eingerichtet ist, die verschiedenen Sensoren zur Durchführung einer reflektiven photoplethysmographischen Messung und einer transmissiven photoplethysmographischen Messung anzusteuern; und
**dadurch gekennzeichnet, dass** die Steuereinheit dazu eingerichtet ist, Ergebnisse der transmissiven photoplethysmographischen Messung zur Validierung der reflektiven photoplethysmographischen Messung zu verwenden.

2. Ringsystem (100) nach Anspruch 1, wobei die Sensoren jeweils dazu eingerichtet sind, eine reflektive photoplethysmographische Messung und gemeinsam mit einem weiteren der Sensoren eine transmissive photoplethysmographische Messung durchzuführen.

3. Ringsystem (100) nach Anspruch 1, wobei die Sensoren (200) jeweils dazu eingerichtet sind, eine reflektive photoplethysmographische Messung an dem Finger eines Benutzers durchzuführen, an dem das Ringsystem getragen wird.

4. Ringsystem (100) nach einem der Ansprüche 1 bis 3, wobei das Ringsystem dazu eingerichtet ist, eine Benutzeridentifikation auf Grundlage von Informationen aus der photoplethysmographischen Messung durchzuführen.

5. Ringsystem (100) nach einem der Ansprüche 1 bis 4, umfassend mindestens 4 der Sensoren (200).

6. Ringsystem (100) nach einem der Ansprüche 1 bis 5, umfassend 8 oder mehr der Sensoren (200).

7. Ringsystem (100) nach einem der Ansprüche 1 bis 6, wobei jeder der Sensoren (200) Photodioden und Lichtemitter umfasst.

8. Ringsystem (100) nach einem der Ansprüche 1 bis 7, wobei jeder der Sensoren (200) eine zweidimensionale Anordnung von Photodioden und Lichtemittern umfasst.

9. Ringsystem (100) nach einem der Ansprüche 1 bis 8, wobei die Sensoren (200) eine ringförmige Photodiodenanordnung und eine ringförmige Lichtemitteranordnung umfassen.

10. Ringsystem (100) nach Anspruch 9, wobei die Lichtemitter organische Leuchtdioden sind und es sich bei der Photodiodenanordnung um organische Photodioden handelt.

11. Ringsystem (100) nach Anspruch 1, wobei die Steuereinheit dazu eingerichtet ist, Ergebnisse der transmissiven Photoplethysmographie zu verwenden, um zu bestimmen, welche der Sensoren (200) für eine fortlaufende reflektive photoplethysmographische Messung verwendet werden.

12. Ringsystem (100) nach einem der Ansprüche 1 bis 11, wobei die Steuereinheit dazu eingerichtet ist, die um den Ring angeordneten Sensoren (200) zur Verfolgung arterieller und/oder venöser Muster zu verwenden.

13. Ringsystem (100) nach einem der Ansprüche 1 bis 12, ferner umfassend einen Sensorblock mit Trägheitssensoren.

14. Ringsystem (100) nach Anspruch 13, wobei der Sensorblock einen Benutzertemperatursensor und einen Umgebungstemperatursensor umfasst.

## Revendications

1. Système de bague (100), comprenant
des capteurs (200) configurés pour effectuer à la fois une mesure photopléthysmographique en transmission et une mesure photopléthysmographique en réflexion sur un doigt d'un utilisateur sur lequel le système de bague est porté ; et
une unité de commande configurée pour commander les différents capteurs afin d'effectuer une mesure photopléthysmographique en réflexion et une mesure photopléthysmographique en transmission ; et
**caractérisé en ce que** l'unité de commande est configurée pour utiliser les résultats de la mesure photopléthysmographique en transmission afin de valider la mesure photopléthysmographique en réflexion.

2. Le système de bague (100) selon la revendication 1, dans lequel les capteurs sont chacun configurés pour effectuer une mesure photopléthysmographique en réflexion et, conjointement avec un autre des capteurs, une mesure photopléthysmographique en transmission.

3. Le système de bague (100) selon la revendication 1, dans lequel les capteurs (200) sont chacun configurés pour effectuer une mesure photopléthysmographique en réflexion sur le doigt d'un utilisateur sur lequel le système de bague est porté.

4. Le système de bague (100) selon l'une quelconque des revendications 1 à 3, dans lequel le système de bague est configuré pour effectuer une identification de l'utilisateur sur la base d'informations issues de la mesure photopléthysmographique.

5. Le système de bague (100) selon l'une quelconque des revendications 1 à 4, comprenant au moins 4 des capteurs (200).

6. Le système de bague (100) selon l'une quelconque des revendications 1 à 5, comprenant 8 ou plus des capteurs (200).

7. Le système de bague (100) selon l'une quelconque des revendications 1 à 6, dans lequel chacun des capteurs (200) comprend des photodiodes et des émetteurs lumineux.

8. Le système de bague (100) selon l'une quelconque des revendications 1 à 7, dans lequel chacun des capteurs (200) comprend un réseau bidimensionnel de photodiodes et d'émetteurs lumineux.

9. Le système de bague (100) selon l'une quelconque des revendications 1 à 8, dans lequel les capteurs (200) comprennent un réseau annulaire de photodiodes et un réseau annulaire d'émetteurs lumineux.

10. Le système de bague (100) selon la revendication 9, dans lequel les émetteurs lumineux sont des diodes électroluminescentes organiques et le réseau de photodiodes est constitué de photodiodes organiques.

11. Le système de bague (100) selon la revendication 1, dans lequel l'unité de commande est configurée pour utiliser les résultats de la mesure photopléthysmographique en transmission afin de déterminer lesquels des capteurs (200) sont utilisés pour une mesure photopléthysmographique en réflexion continue.

12. Le système de bague (100) selon l'une quelconque des revendications 1 à 11, dans lequel l'unité de commande est configurée pour utiliser les capteurs (200) disposés autour de la bague afin de suivre des motifs artériels et/ou veineux.

13. Le système de bague (100) selon l'une quelconque des revendications 1 à 12, comprenant en outre un bloc capteurs comportant des capteurs inertiels.

14. Le système de bague (100) selon la revendication 13, dans lequel le bloc capteurs comprend un capteur de température de l'utilisateur et un capteur de température ambiante.
